# EUROPEAN PATENT SPECIFICATION

(11) **EP 3 813 740 B1**
(45) Date of publication and mention of the grant of the patent: **06.11.2024**
(21) Application number: 19816161.4
(22) Date of filing: 04.06.2019
(51) Int. Cl.: A61F 2/966, A61B 17/12, A61F 2/82

(54) **CORE ASSEMBLY FOR MEDICAL DEVICE DELIVERY SYSTEMS**
KERNANORDNUNG FÜR FREISETZUNGSSYSTEME VON MEDIZINPRODUKTEN
ENSEMBLE NOYAU POUR SYSTÈMES DE POSE DE DISPOSITIF MÉDICAL

(30) Priority: 06.06.2018 US 201816001842
(43) Date of publication of application: 05.05.2021
(73) Proprietor: Covidien LP, Mansfield, MA 02048 (US)
(72) Inventor: NAGESWARAN, Ashok, Irvine, CA 92606 (US)
(74) Representative: Gray, James
(86) International application number: PCT/US2019/035417
(87) International publication number: WO 2019/236597

(56) References cited:
- EP-A1- 1 656 963
- EP-A1- 2 842 525
- EP-A2- 1 344 502
- WO-A1-2012/096687
- WO-A2-97/19713
- US-A1- 2004 092 868
- US-A1- 2007 088 323
- US-A1- 2009 157 048
- US-A1- 2015 066 130
- US-A1- 2015 164 666
- US-A1- 2016 206 454

## Description

### CROSS-REFERENCE TO RELATED APPLICATIONS

The present application claims the benefit of priority to U.S. Patent Application No. 16/001,842, filed June 6, 2018.

### BACKGROUND

Walls of the vasculature, particularly arterial walls, may develop areas of pathological dilatation called aneurysms that often have thin, weak walls that are prone to rupturing. Aneurysms are generally caused by weakening of the vessel wall due to disease, injury, or a congenital abnormality. Aneurysms occur in different parts of the body, and the most common are abdominal aortic aneurysms and cerebral (e.g., brain) aneurysms in the neurovasculature. When the weakened wall of an aneurysm ruptures, it can result in death, especially if it is a cerebral aneurysm that ruptures.

Aneurysms are generally treated by excluding or at least partially isolating the weakened part of the vessel from the arterial circulation. For example, conventional aneurysm treatments include: (i) surgical clipping, where a metal clip is secured around the base of the aneurysm; (ii) packing the aneurysm with small, flexible wire coils (micro-coils); (iii) using embolic materials to "fill" an aneurysm; (iv) using detachable balloons or coils to occlude the parent vessel that supplies the aneurysm; and (v) intravascular stenting.

Intravascular stents are well known in the medical arts for the treatment of vascular stenoses or aneurysms. Stents are prostheses that expand radially or otherwise within a vessel or lumen to support the vessel from collapsing. Methods for delivering these intravascular stents are also well known.

Conventional methods of introducing a compressed stent into a vessel and positioning it within an area of stenosis or an aneurysm include percutaneously advancing a distal portion of a guiding catheter through the vascular system of a patient until the distal portion is proximate the stenosis or aneurysm. A second, inner catheter and a guidewire within the inner catheter are advanced through the distal portion of the guiding catheter. The guidewire is then advanced out of the distal portion of the guiding catheter into the vessel until the distal portion of the guidewire carrying the compressed stent is positioned at the point of the lesion within the vessel. The compressed stent is then released and expanded so that it supports the vessel at the point of the lesion. EP2842525 discloses a stent delivery system that includes an elongate core member sized for insertion into a blood vessel.

### SUMMARY

According to the invention, there is provided a stent delivery system, comprising: a core assembly sized for insertion into a corporeal lumen, the core assembly configured for advancing a stent toward a treatment location in the corporeal lumen, the core assembly comprising: a longitudinally extending tube having a lumen and a helical cut extending along the tube; and an elongate wire extending through the tube lumen, the wire having an intermediate portion disposed distal to the tube; and a stent carried by the intermediate portion, wherein the tube is affixed to the wire at proximal and distal portions of the tube.

Preferably, the wire extends proximal to a proximal end of the tube.

Preferably, a distal portion of the tube is affixed with respect to the wire.

Preferably, a restraint is coupled to the wire, and wherein the distal portion of the tube is welded to the restraint.

Preferably, the system further comprises a catheter having a lumen configured to receive the core assembly therethrough.

Preferably, the tube is sized to substantially fill the lumen of the catheter.

Preferably, the tube fills at least about 80% of the lumen of the catheter.

Preferably, the tube fills at least about 90% of the lumen of the catheter.

Preferably, the proximal and distal portions of the tube are fixed, thereby preventing compression or elongation of the tube.

Preferably, the tube has a wall thickness of between about 50-60 microns.

Preferably, the tube has a longitudinal length of between about 400-600 mm.

Preferably, the wire has a diameter that tapers distally toward a distal end of the wire.

Preferably, a segment of the intermediate portion of the wire has a substantially constant diameter along its length.

Preferably, the wire has a constant-diameter segment that overlaps a distal end of the tube.

Preferably, the constant-diameter segment is between about 3-5 inches (7.6-12.7 cm).

Preferably, the system further comprises a restraint coupled to the wire and affixed to a distal end of tube.

Preferably, the tube is configured to bend preferentially before the wire.

Preferably, the system further comprises a catheter configured to receive the core assembly therethrough, and wherein a bending stiffness of the tube is configured to match a bending stiffness of the catheter.

Preferably, the bending stiffness of the tube is less than 300% of the bending stiffness of the catheter along at least a distal portion of the tube.

Preferably, the distal portion of the tube spans at least 15 inches (38.1 cm) from a distal end of the tube.

Preferably, the distal portion of the tube spans at least 30 inches (76.2 cm) from a distal end of the tube.

Additional features and advantages of the present technology will be set forth in the description below, and in part will be apparent from the description, or may be learned by practice of the subject technology. The advantages of the present technology will be realized and attained by the structure particularly pointed out in the written description and claims hereof as well as the appended drawings.

It is to be understood that both the foregoing general description and the following detailed description are exemplary and explanatory and are intended to provide further explanation of the present technology as claimed.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present technology. For ease of reference, throughout this disclosure identical reference numbers may be used to identify identical or at least generally similar or analogous components or features.
FIG. 1 is a side, cross-sectional view of a medical device delivery system, according to some embodiments.
FIG. 2A is a side view of the core assembly of the medical device delivery system shown in FIG. 1
FIG. 2B is a side, cross-sectional view of the core assembly of FIG. 2A.
FIG. 3 is an enlarged detail view of a portion of the core assembly shown in FIG. 2A.
FIGS. 4 and 5 are enlarged detail views of portions of the core assembly shown in FIG. 2B.
FIGS. 6A and 6B are graphs of bending stiffness of components of a medical device delivery system, according to some embodiments.

### DETAILED DESCRIPTION

Conventional medical device delivery systems may include a core member or core assembly configured to carry a medical device through a catheter. The core assembly can be a single continuous wire, or in some embodiments the core assembly can be a multi-member construction in which a wire segment is connected end-to-end to a tubular segment. For example, the core assembly can include a proximal wire, a tube (e.g., a hypotube) connected at its proximal end to a distal end of the proximal wire, and a distal wire connected at its proximal end to a distal end of the tube. As compared to a solid wire which must be made thinner or of smaller diameter to become more flexible, tubes provide the advantage of having a consistent and relatively larger outer diameter which occupies a greater portion of the catheter lumen, which can reduce kinking or buckling of the catheter along its length in certain use environments. Tubes can also be precisely tailored to have the desired flexibility at different portions along their length, for example, by varying the pitch of a helically extending cut at different segments of the tube, without need to vary or reduce the tube diameter.

However, tubular core assemblies are susceptible to compression and/or elongation during navigation through the catheter. This is particularly true of tubes having one or more helically extending cuts along the length of the tube. Such elongation or compression can disadvantageously alter the performance characteristics of the core assembly, such as flexibility, column strength, and navigability. Additionally, elongation and compression can cause the clinician to lose the desired responsiveness during operation of the delivery system, because movements made by the clinician at the proximal end of the core assembly do not translate in a one-to-one manner with movement of the core assembly in the distal portion of the delivery system.

For example, as the core assembly is advanced distally, one portion of the core assembly may resist movement (for example, due to frictional engagement with an inner wall of the catheter) more than another portion of the core assembly. If a distal portion of the core assembly resists distal movement to a greater degree than a proximal portion of the core assembly, then the distal portion and the proximal portion will move closer towards one another, resulting in compression or buckling of the core assembly and a reduction in its overall length. If, instead, the proximal portion of the core assembly resists proximal movement to a greater degree than the distal portion, then the proximal and distal portions will move further apart, resulting in elongation of the core assembly and an increase in its overall length. In some instances, the tubular core assembly may simultaneously be subject to both compression in some portions and elongation in other portions. Embodiments of the present technology provide a core assembly that offers the benefits of a tubular member while reducing the risk of compression or elongation. For example, the core assembly can include a longitudinally extending wire or shaft, and a tube (e.g., a hypotube having a helically extending cut) surrounding the wire along a portion of the length of the wire. The tube is affixed to the wire at proximal and distal portions of the tube, thereby restricting the ability of the tube to elongate or compress with respect to the wire.

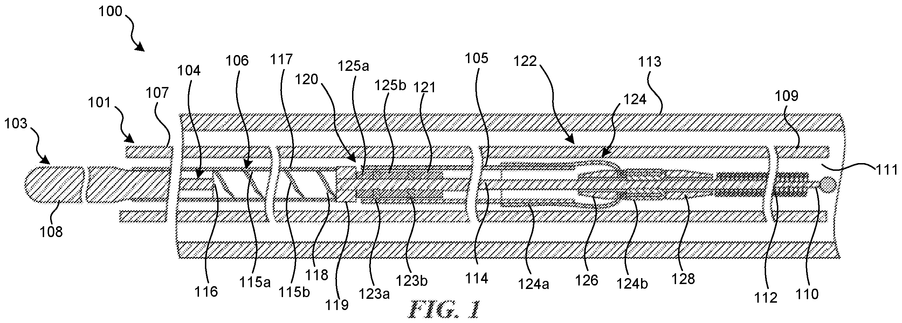

Specific details of several embodiments of the present technology are described herein with reference to FIGS. 1-6B. Although many of the embodiments are described with respect to devices, systems, and methods for delivery of stents and other medical devices, other applications and other embodiments in addition to those described herein are within the scope of the present technology. Further, embodiments of the present technology can have different configurations, components, and/or procedures than those shown or described herein. Moreover, embodiments of the present technology can have configurations, components, and/or procedures in addition to those shown or described herein and these and other embodiments may not have several of the configurations, components, and/or procedures shown or described herein without deviating from the present technology.

As used herein, the terms "distal" and "proximal" define a position or direction with respect to a clinician or a clinician's control device (e.g., a handle of a delivery catheter). For example, the terms, "distal" and "distally" refer to a position distant from or in a direction away from a clinician or a clinician's control device along the length of device. In a related example, the terms "proximal" and "proximally" refer to a position near or in a direction toward a clinician or a clinician's control device along the length of device. The headings provided herein are for convenience only and should not be construed as limiting the subject matter disclosed.

FIGS. 1-5 depict embodiments of medical device delivery systems that may be used to deliver and/or deploy a medical device, such as but not limited to a stent, into a hollow anatomical structure such as a blood vessel. The stent can comprise a braided stent or other form of stent such as a woven stent, knit stent, laser-cut stent, roll-up stent, etc. The stent can optionally be configured to act as a "flow diverter" device for treatment of aneurysms, such as those found in blood vessels including arteries in the brain or within the cranium, or in other locations in the body such as peripheral arteries. The stent can optionally be similar to any of the versions or sizes of the PIPELINE^{™} Embolization Device marketed by Medtronic Neurovascular of Irvine, California USA. The stent can alternatively comprise any suitable tubular medical device and/or other features as described herein. In some embodiments, the stent can be any one of the stents described in U.S. Application No. 15/892,268, filed February 8, 2018, titled VASCULAR EXPANDABLE DEVICES.

FIG. 1 is a side cross-sectional view of a medical device delivery system 100 configured in accordance with an embodiment of the present technology. The delivery system 100 can be configured to carry a stent (or other vascular implant or device) 105 thereon to be advanced through a surrounding elongate tube or catheter 101 to a target site in a patient, for example, a site within a corporeal lumen 113 such as a blood vessel. The catheter 101 can slidably receive a core member or core assembly 103 configured to carry the stent 105 thereon. The depicted catheter 101 has a proximal portion 107 and an opposing distal portion 109 which can be positioned at a treatment site within a patient, and an internal lumen 111 extending from the proximal portion 107 to the distal portion 109. At the distal portion 109, the catheter 101 has a distal opening through which the core assembly 103 may be advanced beyond the distal portion 109 to expand or deploy the stent 105 within the corporeal lumen 113. The proximal portion 107 may include a catheter hub (not shown). The catheter 101 can define a generally longitudinal dimension extending between the proximal portion 107 and the distal portion 109. When the delivery system 100 is in use, the longitudinal dimension need not be straight along some or any of its length.

The delivery system 100 can be used with any number of catheters. For example, the catheter can optionally comprise any of the various lengths of the MARKSMAN^{™} catheter available from Medtronic Neurovascular of Irvine, California USA. The catheter can optionally comprise a microcatheter having an inner diameter of about 0.030 inches (0.76 mm) or less, and/or an outer diameter of 3 French or less near the distal portion. Instead of or in addition to these specifications, the catheter can comprise a microcatheter which is configured to percutaneously access the internal carotid artery, or another location within the neurovasculature distal of the internal carotid artery.

The core assembly 103 can generally comprise any member(s) with sufficient flexibility and column strength to move the stent 105 or other medical device through the catheter 101. The core assembly 103 can therefore comprise a wire, tube (e.g., hypotube), braid, coil, or other suitable member(s), or a combination of wire(s), tube(s), braid(s), coil(s), etc. The embodiment of the core assembly 103 depicted in FIG. 1 is of a multi-member construction, comprising a longitudinally extending shaft or wire 104 and an elongate tube 106 surrounding at least a portion of the wire 104. An outer layer 117, which can comprise a layer of lubricious material such as PTFE (polytetrafluoroethylene or TEFLON^{™}) or other lubricious polymer, can cover some or all of the tube 106 and/or the wire 104.

The wire 104 has a proximal portion 108 and a distal portion 110, which can optionally include a tip coil 112. The wire 104 can be constructed from materials including polymers and metals including nitinol and stainless steels. In some embodiments, the wire 104 tapers in the distal direction, having a larger diameter at the proximal portion 108 and a smaller diameter at the distal portion 110. The taper may be gradual and continuous along the length of the wire 104, or in some embodiments the taper may vary at different portions of the wire 104. As described in more detail below, in some embodiments the wire 104 can include one or more constant-diameter segments in which the wire 104 does not taper. Such constant-diameter segments can be useful for utilizing a single wire in combination with tubes 106 and stents 105 of different lengths.

The wire 104 can also include an intermediate portion 114 located between the proximal portion 108 and the distal portion 110. The intermediate portion 114 includes the portion of the core assembly 103 onto or over which the stent 105 extends when the core assembly 103 is in the pre-deployment configuration as shown in FIG. 1. The wire 104 may include one or more fluorosafe markers (not shown), and such marker(s) can be located on a portion of the wire 104 that is not covered by the outer layer 117 (e.g., proximal of the outer layer 117). This portion of the wire 104 marked by the marker(s), and/or proximal of any outer layer 117, can comprise a bare metal outer surface.

The tube 106 extends from a proximal portion 116 to a distal portion 118 and surrounds the wire 104 along at least a portion of the length of the wire 104. In some embodiments, the distal portion 118 of the tube 106 terminates proximal to the intermediate portion 114 of the wire 104, such that during operation, the stent 105 is carried by the intermediate portion 114 of the wire 104 at a position distal to the tube 106.

The tube 106 can have a sidewall that is "uncut" or without openings or voids formed therein. Alternatively, the tube 106 can have openings, voids, or cuts formed in the sidewall to enhance the flexibility of the tube. This may be done by cutting a series of slots in the sidewall along part or all of the length of the tube, or cutting or drilling a pattern of other openings in the sidewall, or cutting a spiral-shaped void or helical cut in the sidewall. For example, as shown in FIG. 1, the tube 106 has a helically extending void or cut 115 in the sidewall that extends from the proximal portion 116 to the distal portion 118. The cut 115 can include multiple segments having different pitches, for example the first segment 115a has a lower pitch than the second segment 115b. By varying the pitch in different segments of the cut 115, the bending stiffness of the tube 106 can be precisely tailored along the length of the tube 106. Although two segments of the cut 115 are illustrated in FIG. 1, the tube 106 can have a large number of different segments having different pitch dimensions.

In some embodiments, for example where the delivery system is to be used in narrow and/or tortuous vasculature, such as the neurovasculature, the tube 106 can be of relatively small outside diameter (e.g., 0.040" or less, or 0.030" or less, or 0.027" or less, or about 0.020", or about .016"). In some embodiments, the outer diameter of the tube 106 can be configured to substantially fill the lumen of the catheter 101. As used herein, "fill" means that an outer diameter of the tube 106 extends substantially across the internal diameter of the lumen 111 of the catheter 101. In some embodiments, the tube 106 fills at least about 50%, 60%, 70%, 80%, 90%, or more of the lumen of the catheter.

The tube 106 can have a relatively thin sidewall thickness (e.g., 80 microns or less, 70 microns or less, 60 microns or less, or 50 microns or less, or between about 50 and 60 microns). The tube 106 can also have a relatively long overall length (e.g., 400 mm or more, 500 mm or more, 600 mm or more, 700 mm or more, 800 mm or more, between about 400 and 600 mm, or about 514 mm). Instead of or in addition to any one or combination of such dimensions, the tube 106 can have a relatively long cut length (the length of the portion of the tube 106 in which opening(s), void(s), spiral(s), or cut(s) 115 is/are present) of 400 mm or more, 500 mm or more, 600 mm or more, 700 mm or more, 800 mm or more, or about 514 mm.

A relatively long, small-diameter, and/or thin-walled spiral-cut tube offers certain advantages for use in the core assembly 103 in narrow and/or tortuous vasculature, such as the neurovasculature. The tube 106 can be made highly flexible (or inflexible as the case may be) where necessary by use of an appropriate spiral pitch, and the column strength or "pushability" of the tube 106 can be maintained largely independent of its flexibility, as the diameter of the tube 106 can remain constant along its length. The combination of high flexibility and pushability can facilitate easier navigation into difficult, tortuous vascular locations.

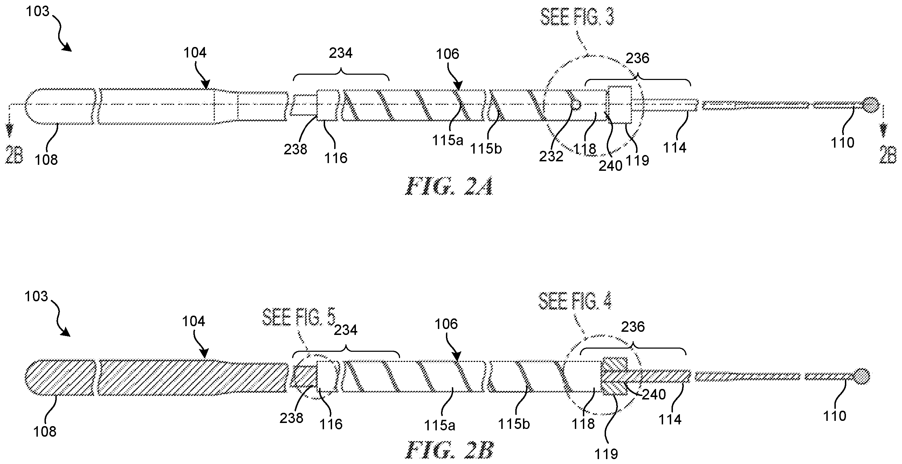

In various embodiments of the tube 106, the helical or spiral cut 115 can be relatively long and continuous. For example, the tube 106 can have such a helical or spiral cut 115 over any of the various cut lengths specified above or elsewhere herein for the tube 106. A tube 106 having such a helical or spiral cut 115 can also have any one or combination of the various outside diameters, sidewall thicknesses, and/or overall lengths specified above or elsewhere herein for the tube 106. The helical or spiral cut can extend along the entire length of the tube, or nearly the entire length, e.g. the entire length except for a small uncut portion at the distal and/or proximal end, as shown in FIG. 2A with regard to the distal end where the spiral cut is terminated with a stress relief hole.

The long contiguous or continuous helical or spiral cut 115 can be implemented using any number of techniques. In one approach, two or more longitudinally adjacent spirals, cuts, slots or voids can be formed contiguously or continuously in the sidewall of the tube 106 and joined at their adjacent ends by connection aperture(s) to form a spiral or helical cut, slot, or void that is contiguous or continuous along the overall length or along the cut length of the tube 106. In some embodiments, the individual spirals, cuts, slots, or voids can be about 150 mm in length, or 150 mm or less in length. These need not be uniform in length along the tube or cut length; for example, the first or last spiral, cut, slot, or void can be made somewhat shorter in order to achieve a cut length that is not an even multiple of the length of the individual spirals.

In some embodiments, one or more terminal apertures may be employed in the spiral or helical cut, slot, or void. In still other embodiments of the tube 106, a spiral or helical cut, slot, or void is employed with terminal aperture(s) at one or both terminal ends and no connecting apertures along the cut length. One or multiple such spirals may be formed in the sidewall of a single tube 106. Where employed, the terminal aperture(s) can serve as a stress relief or measure against sidewall crack formation at the end(s) of the spiral.

Instead of or in addition to a spiral cut 115 that is contiguous or continuous over a relatively long overall length or cut length of the tube 106, the pitch of the spiral can be controlled precisely over a long overall length or cut length. For example, the pitch of the spiral cut 115 can vary over the cut length such that a pitch of a specific magnitude can prevail along a relatively short segment of the cut length, for example 5 mm or less, 3 mm or less, 2 mm or less, or about 1.0 mm. In this manner, the spiral pitch can be finely adjusted in small increments of the cut length thereby facilitating superior control over the mechanical properties of the tube 106 (e.g., bending stiffness, column strength) in various portions of the tube. Therefore, the tube 106 can have a pitch that varies in magnitude (including a specific "first pitch magnitude") along the overall length or cut length of the tube, and the first pitch magnitude can prevail along a first segment of the cut length. The first segment can have a length (measured along the longitudinal dimension of the tube 106) of 5 mm or less, 3 mm or less, 2 mm or less, or about 1.0 mm. The magnitude of the pitch can change from the first magnitude at one or both ends of the first segment. The first segment can be located (e.g., in a contiguous or continuous void) anywhere along the cut length, including location(s) relatively far from the endpoints of the cut length, e.g., more than 100 mm away, more than 200 mm away, or more than 300 mm away from an endpoint of the cut length.

Instead of or in addition to achievement of a particular pitch magnitude in one or more short segments of the cut length (and/or a spiral that is contiguous or continuous over a relatively long overall length or cut length of the tube 106), the pitch magnitude can be controlled precisely so that it can vary in relatively small increments. (The pitch can be expressed in mm/rotation.) For example, the pitch can vary in magnitude by 0.2 mm/rotation or less, 0.1 mm/rotation or less, 0.01 mm/rotation or less, or 0.005 mm/rotation or less. This provides another manner in which the spiral can be finely controlled to facilitate desired mechanical properties in various portions of the tube 106. Therefore, the tube 106 can have a pitch that varies in magnitude (including a specific "first pitch magnitude") along the overall length or cut length of the tube, and the first pitch magnitude can prevail along a first segment of the cut length. The magnitude of the pitch can change from the first magnitude by 0.2 mm/rotation or less, 0.1 mm/rotation or less, 0.01 mm/rotation or less, or 0.005 mm/rotation or less, at one or both ends of the first segment. The first segment can be located (e.g., in a contiguous or continuous void) anywhere along the cut length, including location(s) relatively far from the endpoints of the cut length, e.g., more than 100 mm away, or more than 200 mm away, or more than 300 mm away from an endpoint of the cut length.

As described in more detail below, the tube 106 can be mounted over the wire 104 such that the tube 106 is affixed to the wire 104. For example, the tube 106 can be affixed to the wire 104 at one or more contact points. In one embodiment, the tube 106 is affixed to the wire 104 at one or more contact points in the proximal portion 116 and at one or more contact points in the distal portion 118 of the tube 106. The tube 106 can be affixed to the wire 104 at these contact points by soldering, welding, adhesive, or other suitable fixation technique. In some embodiments, there may be two, three, four, or more contact points at which the tube 106 is affixed to the wire 104. In other embodiments, the tube 106 may be affixed to the wire 104 at only a single contact point. In still other embodiments, the tube 106 may not be affixed to the wire 104. As used herein, "affixed" includes both direct and indirection fixation, for example, the wire 104 can be directly welded or adhered to the tube 106 at a contact point, or the tube can be welded or otherwise attached to an intervening member (e.g., the proximal restraint 119) which in turn is affixed directly to the wire 104.

Affixing portions of the tube 106 to the wire 104 can reduce or eliminate elongation or compression of the tube 106 during operation of the delivery system 100. As noted above, the relatively large diameter of the tube 106 can enhance pushability of the core assembly 103. However, the presence of flexibility enhancing cuts (e.g., the helical cut 115 extending along the length of the tube) may cause the tube 106 to elongate or compress during movement of the core assembly 103 with respect to the catheter 101. For example, during distal advancement of the core assembly 103, the distal portion 118 of the tube 106 may resist movement (for example, due to frictional engagement with an inner wall of the catheter 101) more than the proximal portion 116 of the tube 106. As a result, the proximal portion 116 and the distal portion 118 would move closer towards one another, resulting in compression of the tube 106 and a reduction in overall length. If instead the distal portion 118 of the tube 106 resisted proximal movement to a greater degree than the proximal portion 116, then the proximal portion 116 and the distal portion 118 would move further apart, resulting in elongation of the tube 106 and an increase in its overall length. Both elongation and compression can disadvantageously alter the performance characteristics of the tube 106, and therefore the core assembly 103. For example, elongation or compression can modify the flexibility, column strength, and navigability of the core assembly 103. By affixing the tube 106 to the underlying wire 104 at one or more contact points, the risk of compression or elongation of the tube 106 can be reduced. In some embodiments, proximal and distal ends of the tube 106 can be affixed to the wire 104, thereby effectively fixing the overall length of the tube 106 and substantially eliminating compression or elongation of the tube 106 during operation of the delivery system 100. In other embodiments, the tube 106 can be affixed to the wire 104 at contact points that are spaced apart from proximal and distal ends of the tube 106.

The system 100 can also include a coupling assembly 120 or resheathing assembly 120 configured to releasably retain the medical device or stent 105 with respect to the core assembly 103. The coupling assembly 120 can be configured to engage the stent 105 via mechanical interlock with the pores and filaments of the stent 105, abutment of the proximal end or edge of the stent 105, frictional engagement with the inner wall of the stent 105, or any combination of these modes of action. The coupling assembly 120 can therefore cooperate with the overlying inner surface of the catheter 101 to grip and/or abut the stent 105 such that the coupling assembly 120 can move the stent 105 along and within the catheter 101, e.g., distal and/or proximal movement of the core assembly 103 relative to the catheter 101 results in a corresponding distal and/or proximal movement of the stent 105 within the catheter lumen 111.

The coupling assembly 120 (or portion(s) thereof) can, in some embodiments, be configured to rotate about the core assembly 103. In some such embodiments, the coupling assembly 120 can comprise a proximal bumper or restraint 119 and a distal restraint 121. The proximal and distal restraints 119, 121 can be fixed to the core assembly 103 to prevent or limit proximal or distal movement of the coupling assembly 120 along the longitudinal dimension of the core assembly 103. For example, the proximal and distal restraints 119, 121 can be soldered or fixed with adhesive to the core wire 104. One or both of the proximal and distal restraints 119, 121 can have an outside diameter or other radially outermost dimension that is smaller than the outside diameter or other radially outermost dimension of the overall coupling assembly 120 such that one or both of the restraints 119, 121 do not contact the inner surface of the stent 105 during operation of the system 100. In some embodiments, the proximal restraint 119 can be sized to abut the proximal end of the stent 105 and be employed to push the stent distally during delivery.

The coupling assembly 120 can also include first and second stent engagement members (or device engagement members, or resheathing members) 123a-b (together "engagement members 123") and first and second spacers 125a-b (together "spacers 125") disposed about the core assembly 103 between the proximal and distal restraints 119, 121. In the illustrated embodiment, from proximal to distal, the elements of the coupling assembly 120 include the proximal restraint 119, followed by the first spacer 125a, the first stent engagement member 123a, the second spacer 125b, the second stent engagement member 123b, and finally the distal restraint 121. In this configuration, the first spacer 125a defines the relative positioning of the first engagement member 123a and the proximal restraint 119. The second spacer 125b defines the relative longitudinal spacing between the first engagement member 123a and the second engagement member 123b.

One or both of the spacers 125 can take the form of a wire coil, a solid tube, or other structural element that can be mounted over the core assembly 103 to longitudinally separate adjacent components of the coupling assembly 120. In some embodiments, one or both of the spacers 125 can be a zero-pitch coil with flattened ends. In some embodiments, one or both of the spacers 125 can be a solid tube (e.g., a laser-cut tube) that can be rotatably mounted or non-rotatably fixed (e.g., soldered) to the core assembly 103. The spacers 125 can have a radially outermost dimension that is smaller than a radially outermost dimension of the engagement members 123 such that the spacers 125 do not contact the stent 105 during normal operation of the system 100. The dimensions, construction, and configuration of the spacers 125 can be selected to achieve improved grip between the coupling assembly 120 and the overlying stent 105.

The stent 105 can be moved distally or proximally within the overlying catheter 101 via the proximal coupling assembly 120. In some embodiments, the stent 105 can be resheathed via the proximal coupling assembly 120 after partial deployment of the stent 105 from a distal opening of the catheter. In embodiments in which the proximal restraint 119 is sized to abut the proximal end of the stent 105 and employed to push the stent distally during delivery, the first and second stent engagement members 123a-b can be employed to resheath the stent 105 after partial deployment, while taking no (or substantially no) part in pushing the stent distally during delivery. For example, the first and second stent engagement members 123a-b can in such embodiments transmit no, or substantially no, distal push force to the stent 105 during delivery.

Optionally, the proximal edge of the proximal coupling assembly 120 can be positioned just distal of the proximal edge of the stent 105 when in the delivery configuration. In some such embodiments, this enables the stent 105 to be re-sheathed when as little as a few millimeters of the stent remains in the catheter. Therefore, with stents of typical length, resheathability of 75% or more can be provided (i.e. the stent can be re-sheathed when 75% or more of it has been deployed).

With continued reference to FIG. 1, the distal interface assembly 122 can comprise a distal engagement member 124 that can take the form of, for example, a distal device cover or distal stent cover (generically, a "distal cover"). The distal cover 124 can be configured to reduce friction between the stent 105 (e.g., a distal portion thereof) and the inner surface of the surrounding catheter 101. For example, the distal cover 124 can be configured as a lubricious, flexible structure having a free first end or section 124a that can extend over at least a portion of the stent 105 and/or intermediate portion 108 of the core assembly 103, and a fixed second end or section 124b that can be coupled (directly or indirectly) to the core assembly 103.

The distal cover 124 can have a first or delivery position, configuration, or orientation in which the distal cover can extend proximally relative to the distal tip, or proximally from the second section 124b or its (direct or indirect) attachment to the core assembly 103, and at least partially surround or cover a distal portion of the stent 105. The distal cover 124 can be movable from the first or delivery orientation to a second or resheathing position, configuration, or orientation (not shown) in which the distal cover can be everted such that the first end 124a of the distal cover is positioned distally relative to the second end 124b of the distal cover 124 to enable the resheathing of the core assembly 103, either with the stent 105 carried thereby, or without the stent 105. As shown in FIG. 1, the first section 124a of the distal cover 124 can originate from the proximal end of the second section 124b. In another embodiment, the first section 124a can originate from the distal end of the second section 124b.

The distal cover 124 can be manufactured using a lubricious and/or hydrophilic material such as PTFE or Teflon^{®}, but may be made from other suitable lubricious materials or lubricious polymers. The distal cover can also comprise a radiopaque material which can be blended into the main material (e.g., PTFE) to impart radiopacity. The distal cover 124 can have a thickness of between about 0.0005" and about 0.003' (one inch = 25,4mm). In some embodiments, the distal cover can be one or more strips of PTFE having a thickness of about 0.001".

The distal cover 124 (e.g., the second end 124b thereof) can be fixed to the core assembly 103 (e.g., to the wire 104 or distal tip thereof) so as to be immovable relative to the core assembly 103, either in a longitudinal/sliding manner or a radial/rotational manner. Alternatively, as depicted in FIG. 1, the distal cover 124 (e.g., the second end 124b thereof) can be coupled to (e.g., mounted on) the core assembly 103 so that the distal cover 124 can rotate about a longitudinal axis of the core assembly 103 (e.g., of the wire 104), and/or move or slide longitudinally along the core assembly 103. In such embodiments, the second end 124b can have an inner lumen that receives the core assembly 103 therein such that the distal cover 124 can slide and/or rotate relative to the core assembly 103. Additionally, in such embodiments, the distal interface assembly 122 can further comprise a proximal restraint 126 that is fixed to the core assembly 103 and located proximal of the (second end 124b of the) distal cover 124, and/or a distal restraint 128 that is fixed to the core assembly 103 and located distal of the (second end 124b of the) distal cover 124. The distal interface assembly 122 can comprise a radial gap between the outer surface of the core assembly 103 (e.g., of the wire 104) and the inner surface of the second end 124b. Such a radial gap can be formed when the second end 124b is constructed with an inner luminal diameter that is somewhat larger than the outer diameter of the corresponding portion of the core assembly 103. When present, the radial gap allows the distal cover 124 and/or second end 124b to rotate about the longitudinal axis of the core assembly 103 between the restraints 126, 128.

In some embodiments, one or both of the proximal and distal restraints 126, 128 can have an outside diameter or other radially outermost dimension that is smaller than the (e.g., pre-deployment) outside diameter or other radially outermost dimension of the distal cover 124, so that one or both of the restraints 126, 128 will tend not to bear against or contact the inner surface of the catheter during operation of the core assembly 103. Alternatively, it can be preferable to make the outer diameters of the restraints 126 and 128 larger than the largest radial dimension of the pre-deployment distal cover 124, and/or make the outer diameter of the proximal restraint 126 larger than the outer diameter of the distal restraint 128. This configuration allows easy and smooth retrieval of the distal cover 124 and the restraints 126, 128 back into the catheter post stent deployment.

In operation, the distal cover 124, and in particular the first section 124a, can generally cover and protect a distal portion of the stent 105 as the stent 105 is moved distally through a surrounding catheter. The distal cover 124 may serve as a bearing or buffer layer that, for example, inhibits filament ends of the distal portion of the stent 105 (where the stent comprises a braided stent) from contacting an inner surface of the catheter, which could damage the stent 105 and/or catheter, or otherwise compromise the structural integrity of the stent 105. Since the distal cover 124 may be made of a lubricious material, the distal cover 124 may exhibit a low coefficient of friction that allows the distal portion of the stent to slide axially within the catheter with relative ease. The coefficient of friction between the distal cover 124 and the inner surface of the catheter 101 can be between about 0.02 and about 0.4. For example, in embodiments in which the distal cover and the catheter are formed from PTFE, the coefficient of friction can be about 0.04. Such embodiments can advantageously improve the ability of the core assembly 103 to pass through the catheter, especially in tortuous vasculature.

Structures other than the herein-described embodiments of the distal cover 124 may be used in the core assembly 103 and/or distal interface assembly 122 to cover or otherwise interface with the distal portion of the stent 105. For example, a protective coil or other sleeve having a longitudinally oriented, proximally open lumen may be employed. In other embodiments, the distal interface assembly 122 can omit the distal cover 124, or the distal cover can be replaced with a component similar to the proximal coupling assembly 120. Where the distal cover 124 is employed, it can be connected to the distal tip coil 112 (e.g., by being wrapped around and enclosing some or all of the winds of the coil 112) or being adhered to or coupled to the outer surface of the coil by an adhesive or a surrounding shrink tube. The distal cover 124 can be coupled (directly or indirectly) to other portions of the core assembly 103, such as the wire 104.

In embodiments of the core assembly 103 that employ both a rotatable proximal coupling assembly 120 and a rotatable distal cover 124, the stent 105 can be rotatable with respect to the core assembly 103 about the longitudinal axis thereof, by virtue of the rotatable connections of the proximal coupling assembly 120 and distal cover 124. In such embodiments, the stent 105, proximal coupling assembly 120, and distal cover 124 can rotate together in this manner about the core assembly 103. When the stent 105 can rotate about the core assembly 103, the core assembly 103 can be advanced more easily through tortuous vessels as the tendency of the vessels to twist the stent 105 and/or core assembly 103 is negated by the rotation of the stent 105, proximal coupling assembly 120, and distal cover 124 about the core assembly 103. In addition, the required push force or delivery force is reduced, as the user's input push force is not diverted into torsion of the stent 105 and/or core assembly 103. The tendency of a twisted stent 105 and/or core assembly 103 to untwist suddenly or "whip" upon exiting tortuosity or deployment of the stent 105, and the tendency of a twisted stent to resist expansion upon deployment, are also reduced or eliminated. Further, in some such embodiments of the core assembly 103, the user can "steer" the core assembly 103 via the tip coil 112, particularly if the coil 112 is bent at an angle in its unstressed configuration. Such a coil tip can be rotated about a longitudinal axis of the system 100 relative to the stent 105, coupling assembly 120 and/or distal cover 124 by rotating the distal portion 110 of the core assembly 103. Thus the user can point the coil tip 112 in the desired direction of travel of the core assembly 103, and upon advancement of the core assembly the tip will guide the core assembly in the chosen direction.

FIG. 2A is a side view of the core assembly 103 of the medical device delivery system 100 shown in FIG. 1, and FIG. 2B is a side cross-sectional view of the core assembly 103 of FIG. 2A taken along line 2B-2B. As noted above, the core assembly 103 includes an elongate shaft or wire 104 and a longitudinally extending tube 106 that surrounds the wire 104 along at least a portion of the length of the wire 104. The wire 104 includes a proximal portion 108, a distal portion 110, and an intermediate portion 114 configured to carry the stent 105 (FIG. 1) thereon. The tube 106 is disposed over the wire 104 such that the wire 104 extends through an inner lumen of the tube 106. The wire 104 can have a length greater than a length of the tube 106, such that the wire 104 extends proximal to the proximal portion 116 of the tube 106 and also extends distal to the distal portion 118 of the tube 106.

As noted above, the tube 106 can have a spiral or helical void or cut 115 extending along at least a portion of the length of the tube 106, and the cut 115 can include one or more segments 115a, 115b having different pitch dimensions to impart varying bending stiffness to different portions of the tube 106 along its length. The cut 115 can terminate in an aperture 232 formed in the sidewall of the tube 106. The aperture 232 can comprise an additional void that is formed (e.g., cut) in the sidewall of the tube 106 and is contiguous or continuous with the void or cut 115. The aperture 232 can comprise a circle, as shown in FIG. 2A, or any other suitable shape such as an ellipse or polygon. When employed, the aperture 232 can serve as a stress relief or measure against sidewall crack formation at the end of the helical cut 115. In some embodiments, different segments of the cut 115 (e.g., first segment 115a and second segment 115b) can be connected by connection apertures, thereby forming a single, contiguous, or continuous void. The connection apertures can be substantially similar to the aperture 232, except that they are positioned between adjacent segments of the cut 115 (e.g., between the first segment 115a and the second segment 115b).

The wire 104 can have an outer profile that tapers radially inwardly in the distal direction, having a larger outer profile (e.g., diameter) at the proximal portion 108 and a smaller outer profile at the distal portion 110. The taper may be gradual and continuous along the length of the wire 104, or in some embodiments the taper may vary at different portions of the wire 104. In the embodiment illustrated in FIGS. 2A and 2B, the wire 104 can include two (or more) constant-diameter segments: a first constant-diameter segment 234 and a second constant-diameter segment 236. Over each of these segments 234, 236, the wire 104 can have a substantially uniform (i.e., non-tapered) outer profile. The first constant-diameter segment 234 can be positioned to underlie the proximal portion 116 of the tube 106, and the second constant-diameter segment 236 can be positioned to underlie the distal portion 118 of the tube 106. Because the wire 104 tapers distally from the first constant-diameter segment 234 to the second constant-diameter segment 236, in some embodiments the outer profile (e.g., diameter) of the first constant-diameter segment 234 is greater than the outer profile (e.g., diameter) of the second constant-diameter segment 236. In some embodiments, the first constant-diameter segment 234 can have a length of between about 1" to 8", 2" to 6", 3" to 5", or about 4". In some embodiments, the second constant-diameter segment 236 can likewise have a length of between about 1" to 8", 2" to 6", 3" to 5", or about 4".

These first and second constant-diameter segments 234 and 236 can provide portions of the wire 104 configured to be affixed to corresponding portions of the surrounding tube 106. The tube 106 can be affixed to the wire 104 at a first contact point 238 at the proximal portion 116 of the tube 106, and can also be affixed to the wire 104 at the second contact point 240 at the distal portion 118 of the tube 106. The first contact point 238 can be positioned at any longitudinal position within the first constant-diameter segment 234, and the second contact point 240 can be positioned at any longitudinal position within the second constant-diameter segment 236. Accordingly, the first and second constant-diameter segments 234, 236 enable the wire 104 to accommodate the first and second contact points 238, 240 at a range of different longitudinal positions within the first and second constant-diameter segments, 234, 236, respectively. For example, in various embodiments, the first contact point 238 can be positioned at any longitudinal position along the length of the first constant-diameter segment 234, and the second contact point 240 can be positioned at any longitudinal position along the length of the second constant-diameter segment 236.

This feature can be useful for utilizing a single configuration of the wire 104 in combination with tubes 106 and/or stents 105 (FIG. 1) of different lengths. For example, the intermediate portion 114 of the wire 104 may accommodate stents having a range of different stent sizes. In the case of longer stents, the proximal end of the stent may extend more proximally along the wire than with a shorter stent. To position the proximal bumper or restraint 119 adjacent to the proximal end of the stent, the restraint 119 may be placed at different longitudinal positions along the second constant-diameter segment 236 depending on the length and position of the stent. With a shorter stent, the restraint 119 (along with the distal portion 118 of the tube 106) will be positioned more distally than with a longer stent. By moving the restraint 119 and the distal portion 118 of the tube 106 along the second constant-diameter segment 236, the proximal portion 116 of the tube 106 is also moved along the first constant-diameter segment 234 by an equivalent amount. Accordingly, the lengths of the first and second constant-diameter segments 234, 236 can provide a range of longitudinal positions over which the first and second contact points 238, 240 can be located.

The wire 104 and the tube 106 can be configured such that, during operation of the delivery system, the tube 106 preferentially bends before the wire 104. For example, the sidewall thickness, material section, and helical cut 115 of the tube 106 can all be varied to provide the desired bending stiffness at different portions along the length of the tube 106. Likewise, the material and dimensions of the wire 104 can be varied along the length of the wire 104 to provide varied bending stiffness along its length. The relative bending stiffnesses of the wire 104 and the tube 106 can be configured such that, when bending the core assembly 103 (for example, during navigation of tortuous anatomy), the tube 106 bends before the wire 104. This allows strain to be borne primarily by the tube 106, which can reduce the load borne by the wire 104 and decrease the required delivery force.

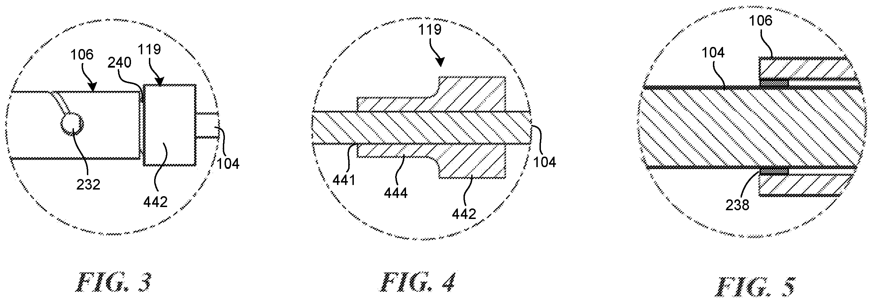

As noted above, the tube 106 can be affixed to the wire 104 along the first constant-diameter segment 234 at a first contact point 238. As seen best in FIG. 5, the first contact point 238 can be at the proximalmost end of the tube 106. The wire 104 can be affixed to the tube 106 at the first contact point 238 via welding, soldering, adhesive, or any other suitable fixation technique. The outer profile (e.g., diameter) of the wire 104 can be configured to facilitate fixation of the wire 104 to the tube 106 at the first contact point 238. For example, in some embodiments, the outer profile of the wire 104 is nearly as large as the inner profile of the lumen of the tube 106, such that the wire 104 substantially fills the lumen of the tube 106 at the first contact point 238. This can facilitate welding, soldering, or otherwise affixing or attaching the wire 104 to the tube 106. As the outer profile of the tube 106 can be substantially constant along the first constant-diameter segment 234, the tube 106 can be affixed to the wire 104 at the first contact point 238 at any point along the length of the first constant-diameter segment 234.

The tube 106 can be affixed to the restraint 119 along the second constant-diameter segment 236 at a second contact point 240. As best seen in FIGS. 3 and 4, the proximal bumper or restraint 119 can be mounted over the wire 104 at a longitudinal position within the second constant-diameter segment 236 of the wire 104. The restraint 119 includes an inner lumen 441 configured to receive the wire 104 therethrough. The lumen 441 can be sized to correspond to an outer profile (e.g., diameter) of the wire 104 along the second constant-diameter segment 236. In some embodiments, the restraint 119 is welded, soldered, or otherwise fixed with respect to the wire 104 such that it cannot rotate or translate with respect to the wire 104. In other embodiments, the restraint 119 can be configured to permit rotation and/or translation within a predefined range with respect to the wire 104.

The restraint 119 further includes a distal section 442 having a first outer profile and a proximal section 444 having a second outer profile that is less than the first outer profile. The outer profile of the proximal section 444 can be sized and configured to fit within the lumen of the tube 106, while the outer profile of the distal section 442 can be sized and configured to abut the distal end of the tube 106. In some embodiments, the outer profile of the distal section 442 matches or exceeds the outer profile of the tube 106, such that the distal portion 118 of the tube 106 cannot move distally beyond the distal section 442 of the restraint 119. When the tube 106 is positioned over the proximal section 444 of the restraint 119 (as seen in FIG. 3), the proximal section 444 can partially overlap the aperture 232 within the sidewall of the tube 106. In some embodiments, the proximal section 444 does not overlap the aperture 232 by more than 30%, more than 40%, more than 50%, more than 60%, or more than 70%. In some embodiments, the proximal section 444 does not overlap the aperture 232 at all.

The tube 106 can be affixed to the restraint 119 at the second contact point 240 via welding, soldering, adhesive, or any other suitable fixation technique. The outer profile (e.g., diameter) of the restraint 119 can be configured to facilitate fixation of the restraint 119 to the tube 106 at the second contact point 240. For example, in some embodiments, the outer profile of the proximal section 444 of the restraint 119 is nearly as large as the inner profile of the lumen of the tube 106, such that the proximal section 444 of the restraint 119 substantially fills the lumen of the tube 106 at the second contact point 240. This can facilitate welding, soldering, or otherwise affixing or attaching the restraint 119 to the tube 106.

In some embodiments, the tube 106 can be affixed directly to the wire 104 at the second contact point 240, for example via welding, soldering, adhesive, or other fixation technique. In some embodiments, instead of the restraint 119, the tube 106 can be connected to another intervening member which in turn is attached to the wire 104. For example, an attachment member separate from the restraint 119 can be affixed to the wire 104, and the tube 106 can in turn be affixed to the attachment member at the second contact point 240.

In some embodiments, the wire 104 may include only the first constant-diameter segment 234 and omit the second constant-diameter segment 236, while in other embodiments the wire 104 may include only the second constant-diameter segment 236 and omit the first constant-diameter segment. In still other embodiments, the wire 104 may omit both the first and second constant-diameter segments 234, 236, instead having a tapering or otherwise varying outer profile in those segments of the wire 104.

Although the first and second contact points 238, 240 are shown as being at or near proximal and distal ends of the tube 106, in some embodiments one or more of the first and second contact points can be located at positions spaced apart from the proximal and distal ends of the tube 106. Additionally or alternatively, in some embodiments there may be additional contact points located at other longitudinal locations along the wire. For example, an additional contact point can be provided between the first and second contact points, thereby providing another point of fixation between the tube and the wire and further preventing compression or elongation of the tube with respect to the wire.

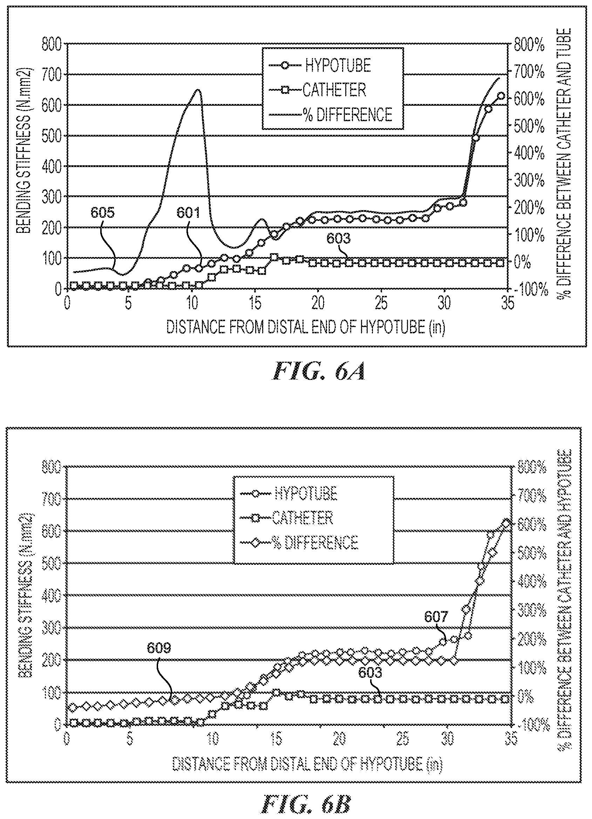

FIGS. 6A and 6B are graphs of bending stiffness of components of a medical device delivery system, according to some embodiments. Referring to FIG. 6A, line 601 depicts the bending stiffness of a hypotube as a function of distance from its distal end. The bending stiffness generally increases in the proximal direction, such that the distalmost portion (on the left side of the graph) has the lowest bending stiffness, and the proximalmost portion (on the right side of the graph) has the highest bending stiffness. The bending stiffness can increase in a series of step changes. Line 603 depicts the bending stiffness of a catheter configured to receive the hypotube therethrough. As with the hypotube, the bending stiffness of the catheter increases with distance from the distal end. However, the catheter has a smaller increase in bending stiffness across its length. Line 605 depicts the difference in bending stiffness at each point between the hypotube and the catheter, expressed as a percentage. High degrees of mismatch between the bending stiffness of the catheter and the hypotube can contribute to increased kinking of the catheter, especially when navigating tortuous anatomy such as the neurovasculature. As shown in FIG. 6A, there is a large increase in line 605 between 5 and 15 inches (127 and 381 mm), reflecting the large difference in bending stiffness between the catheter and the hypotube over this length range. This can disadvantageously lead to kinking or other obstructions that might increase the overall delivery force required to advance the core member or hypotube through the catheter.

To ameliorate these problems, the bending stiffness of the hypotube and/or the catheter can be modified to reduce the difference between the two over at least a portion of their lengths. FIG. 6B illustrates bending stiffness of components of a delivery system in which the hypotube has been modified to have decreased bending stiffness in the range of 5 to 15 inches (127 and 381 mm) compared to the hypotube of FIG. 6A. Referring to FIG. 6B, line 603 again reflects the bending stiffness of the catheter, which is unchanged relative to FIG. 6A. Line 607 depicts the bending stiffness of the hypotube, which is reduced in the range of 5 to 15 inches (127 and 381 mm) relative to FIG. 6A. Line 609 depicts the percentage difference in bending stiffness between the hypotube and the catheter. As seen in FIG. 6B, line 609 remains low along the 5 to 15 inch (127 and 381 mm) range, reflecting similar bending stiffnesses of the hypotube and the catheter in this range as compared to FIG. 6A. Accordingly, by reducing the bending stiffness of the tube in the 5 to 15 inch (127 and 381 mm) range, the bending stiffness of the hypotube is more nearly matched to that of the catheter. Delivery and resheathing forces were evaluated for the designs illustrated in FIGS. 6A and 6B, and it was found that the design of FIG. 6B resulted in a 21% reduction in delivery force and a 32% drop in resheathing force compared to the design of FIG. 6A. This demonstrates that delivery system performance can be improved by more closely matching the bending stiffness of the catheter to the bending stiffness of the hypotube along at least a portion of their lengths.

To more closely match the bending stiffness of the tube to the bending stiffness of the catheter, the tube can be configured to have a bending stiffness that is less than 500%, less than 400%, less than 300%, or less than 200% of the bending stiffness of the catheter along at least a distal portion of the tube. In some embodiments, the distal portion of the hypotube spans at least 5 inches (127 mm), at least 10 inches (254 mm), at least 15 inches (381 mm), at least 20 inches (508 mm), at least 25 inches (635 mm), or at least 30 inches (762 mm) from a distal end of the hypotube.

### Conclusion

This disclosure is not intended to be exhaustive or to limit the present technology to the precise forms disclosed herein. Although specific embodiments are disclosed herein for illustrative purposes, various equivalent modifications are possible without deviating from the present technology, as those of ordinary skill in the relevant art will recognize. In some cases, well-known structures and functions have not been shown and/or described in detail to avoid unnecessarily obscuring the description of the embodiments of the present technology. Although steps of methods may be presented herein in a particular order, in alternative embodiments the steps may have another suitable order. Similarly, certain aspects of the present technology disclosed in the context of particular embodiments can be combined or eliminated in other embodiments. Furthermore, while advantages associated with certain embodiments may have been disclosed in the context of those embodiments, other embodiments can also exhibit such advantages, and not all embodiments need necessarily exhibit such advantages or other advantages disclosed herein to fall within the scope of the present technology. Accordingly, this disclosure and associated technology can encompass other embodiments not expressly shown and/or described herein.

Throughout this disclosure, the singular terms "a," "an," and "the" include plural referents unless the context clearly indicates otherwise. Similarly, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the terms "comprising" and the like are used throughout this disclosure to mean including at least the recited feature(s) such that any greater number of the same feature(s) and/or one or more additional types of features are not precluded. Directional terms, such as "upper," "lower," "front," "back," "vertical," and "horizontal," may be used herein to express and clarify the relationship between various elements. It should be understood that such terms do not denote absolute orientation. Reference herein to "one embodiment," "an embodiment," or similar formulations means that a particular feature, structure, operation, or characteristic described in connection with the embodiment can be included in at least one embodiment of the present technology. Thus, the appearances of such phrases or formulations herein are not necessarily all referring to the same embodiment. Furthermore, various particular features, structures, operations, or characteristics may be combined in any suitable manner in one or more embodiments.

## Claims

1. A stent delivery system (100), comprising:
a core assembly (103) sized for insertion into a corporeal lumen, the core assembly (103) configured for advancing a stent (105) toward a treatment location in the corporeal lumen, the core assembly (103) comprising:
a longitudinally extending tube (106) having a lumen and a helical cut (115) extending along the tube (106); and
an elongate wire (104) extending through the tube lumen, the wire (104) having an intermediate portion disposed distal to the tube (106); and
a stent (105) carried by the intermediate portion, wherein the tube (106) is affixed to the wire (104) at proximal (116) and distal (118) portions of the tube (106).

2. The system (100) of claim 1, wherein the wire (104) extends proximal to a proximal end of the tube (106).

3. The system (100) of claim 1, wherein a proximal portion of the tube (106) is affixed to the wire (104).

4. The system (100) of claim 3, wherein the proximal portion of the tube (106) is welded to the wire (104).

5. The system (100) of claim 1, wherein a distal portion of the tube is affixed with respect to the wire (104).

6. The system (100) of claim 5, wherein a restraint is coupled to the wire (104), and wherein the distal portion of the tube (106) is welded to the restraint.

7. The system (100) of claim 1, wherein the system further comprises a catheter (101) having a lumen configured to receive the core assembly (103) therethrough.

8. The system (100) of claim 7, wherein the tube (106) is sized to substantially fill the lumen of the catheter (101).

9. The system (100) of claim 1, wherein proximal and distal portions of the tube (106) are fixed, thereby preventing compression or elongation of the tube (106).

10. The system (100) of claim 1, wherein the wire (104) has a diameter that tapers distally toward a distal end of the wire (104).

11. The system (100) of claim 1, wherein the tube (106) is configured to bend preferentially before the wire (104).

12. The system (100) of claim 1, further comprising a catheter (101) configured to receive the core assembly (103) therethrough, and wherein a bending stiffness of the tube (106) is configured to match a bending stiffness of the catheter (101).

13. The system (100) of claim 12, wherein the bending stiffness of the tube (106) is less than 300% of the bending stiffness of the catheter (101) along at least a distal portion of the tube (106).

## Patentansprüche

1. Stentabgabesystem (100), umfassend:
eine Kernanordnung (103), die für eine Einführung in ein Körperlumen bemessen ist, wobei die Kernanordnung (103) für ein Vorschieben eines Stents (105) in Richtung einer Behandlungsstelle in dem Körperlumen konfiguriert ist, die Kernanordnung (103) umfassend:
ein sich in Längsrichtung erstreckendes Rohr (106), das ein Lumen und einen spiralförmigen Schnitt (115), der sich entlang des Rohrs (106) erstreckt, aufweist; und
einen länglichen Draht (104), der sich durch das Rohrlumen erstreckt, wobei der Draht (104) einen Zwischenabschnitt, der zu dem Rohr (106) distal angeordnet ist, aufweist; und
einen Stent (105), der durch den Zwischenabschnitt getragen wird, wobei das Rohr (106) an proximalen (116) und distalen (118) Abschnitten des Rohrs (106) an dem Draht (104) befestigt ist.

2. System (100) nach Anspruch 1, wobei sich der Draht (104) zu einem proximalen Ende des Rohrs (106) proximal erstreckt.

3. System (100) nach Anspruch 1, wobei ein proximaler Abschnitt des Rohrs (106) an dem Draht (104) befestigt ist.

4. System (100) nach Anspruch 3, wobei der proximale Abschnitt des Rohrs (106) mit dem Draht (104) verschweißt ist.

5. System (100) nach Anspruch 1, wobei ein distaler Abschnitt des Rohrs hinsichtlich des Drahts (104) befestigt ist.

6. System (100) nach Anspruch 5, wobei eine Rückhaltevorrichtung mit dem Draht (104) gekoppelt ist, und wobei der distale Abschnitt des Rohrs (106) mit der Rückhaltevorrichtung verschweißt ist.

7. System (100) nach Anspruch 1, wobei das System ferner einen Katheter (101) umfasst, der ein Lumen, das konfiguriert ist, um die Kernanordnung (103) dahindurch aufzunehmen, aufweist.

8. System (100) nach Anspruch 7, wobei das Rohr (106) bemessen ist, um das Lumen des Katheters (101) im Wesentlichen zu füllen.

9. System (100) nach Anspruch 1, wobei proximale und distale Abschnitte des Rohrs (106) fixiert sind, wobei dadurch eine Kompression oder eine Verlängerung des Rohrs (106) verhindert wird.

10. System (100) nach Anspruch 1, wobei der Draht (104) einen Durchmesser, der sich in Richtung eines distalen Endes des Drahts (104) distal verjüngt, aufweist.

11. System (100) nach Anspruch 1, wobei das Rohr (106) konfiguriert ist, um sich vorzugsweise vor dem Draht (104) zu biegen.

12. System (100) nach Anspruch 1, ferner umfassend einen Katheter (101), der konfiguriert ist, um die Kernanordnung (103) dahindurch aufzunehmen, und wobei eine Biegesteifigkeit des Rohrs (106) konfiguriert ist, um einer Biegesteifigkeit des Katheters (101) zu entsprechen.

13. System (100) nach Anspruch 12, wobei die Biegesteifigkeit des Rohrs (106) entlang mindestens eines distalen Abschnitts des Rohrs (106) weniger als 300 % der Biegesteifigkeit des Katheters (101) beträgt.

## Revendications

1. Système de pose d'endoprothèse (100), comprenant :
un ensemble noyau (103) dimensionné pour une insertion dans une lumière corporelle, l'ensemble noyau (103) étant conçu pour faire avancer une endoprothèse (105) vers un point de traitement dans la lumière corporelle, l'ensemble noyau (103) comprenant :
un tube (106) s'étendant longitudinalement ayant une lumière et une coupe hélicoïdale (115) s'étendant le long du tube (106) ; et
un fil allongé (104) s'étendant à travers la lumière du tube, le fil (104) ayant une partie intermédiaire disposée distalement par rapport au tube (106) ; et
une endoprothèse (105) portée par la partie intermédiaire, dans lequel le tube (106) est fixé au fil (104) au niveau des parties proximale (116) et distale (118) du tube (106).

2. Système (100) selon la revendication 1, dans lequel le fil (104) s'étend de manière proximale à une extrémité proximale du tube (106).

3. Système (100) selon la revendication 1, dans lequel une partie proximale du tube (106) est fixée au fil (104).

4. Système (100) selon la revendication 3, dans lequel la partie proximale du tube (106) est soudée au fil (104).

5. Système (100) selon la revendication 1, dans lequel une partie distale du tube est fixée par rapport au fil (104).

6. Système (100) selon la revendication 5, dans lequel un dispositif de retenue est accouplé au fil (104), et dans lequel la partie distale du tube (106) est soudée au dispositif de retenue.

7. Système (100) selon la revendication 1, dans lequel le système comprend en outre un cathéter (101) ayant une lumière conçue pour recevoir l'ensemble noyau (103) à travers celui-ci.

8. Système (100) selon la revendication 7, dans lequel le tube (106) est dimensionné pour remplir sensiblement la lumière du cathéter (101).

9. Système (100) selon la revendication 1, dans lequel les parties proximale et distale du tube (106) sont fixées, empêchant ainsi la compression ou l'allongement du tube (106).

10. Système (100) selon la revendication 1, dans lequel le fil (104) a un diamètre qui s'amenuise de manière distale vers une extrémité distale du fil (104).

11. Système (100) selon la revendication 1, dans lequel le tube (106) est conçu pour se plier de préférence avant le fil (104).

12. Système (100) selon la revendication 1, comprenant en outre un cathéter (101) conçu pour recevoir l'ensemble noyau (103) à travers celui-ci, et dans lequel une rigidité de flexion du tube (106) est conçue pour correspondre à une rigidité de flexion du cathéter (101).

13. Système (100) selon la revendication 12, dans lequel la rigidité de flexion du tube (106) est inférieure à 300 % de la rigidité de flexion du cathéter (101) le long d'au moins une partie distale du tube (106).
